**Europäisches Patentamt**

**European Patent Office**

**Office européen des brevets**

(19)

(11) **EP 1 082 282 B1**

(12) **EUROPÄISCHE PATENTSCHRIFT**

(45) Veröffentlichungstag und Bekanntmachung des Hinweises auf die Patenterteilung:
**18.06.2003 Patentblatt 2003/25**

(21) Anmeldenummer: **99915756.3**

(22) Anmeldetag: **12.04.1999**

(51) Int Cl.⁷: **C07C 29/149**, C07C 31/20

(86) Internationale Anmeldenummer:
**PCT/EP99/02448**

(87) Internationale Veröffentlichungsnummer:
**WO 99/055653 (04.11.1999 Gazette 1999/44)**

(54) **VERFAHREN ZUR HERSTELLUNG VON HEXANDIOL**

METHOD FOR PRODUCING HEXANEDIOL

PROCEDE DE PRODUCTION D'HEXANEDIOL

(84) Benannte Vertragsstaaten:
**BE CH DE ES FR GB IT LI**

(30) Priorität: **23.04.1998 DE 19818096**

(43) Veröffentlichungstag der Anmeldung:
**14.03.2001 Patentblatt 2001/11**

(73) Patentinhaber: **BASF AKTIENGESELLSCHAFT**
**67056 Ludwigshafen (DE)**

(72) Erfinder:
- **BREITSCHEIDEL, Boris**
  **D-67117 Limburgerhof (DE)**
- **PINKOS, Rolf**
  **D-67098 Bad Dürkheim (DE)**
- **STEIN, Frank**
  **D-67098 Bad Dürkheim (DE)**

(56) Entgegenhaltungen:
**WO-A-97/31882**          **US-A- 5 614 644**

EP 1 082 282 B1

**Beschreibung**

Die Erfindung betrifft ein Verfahren zur Herstellung von 1,6-Hexandiol durch Hydrierung von Adipinsäureestern oder Estergemischen, die diese enthalten und die organische Halogenverbindungen als Verunreinigung enthalten, wobei man das Edukt vor der Hydrierung über einen Kupferkatalysator zur Entfernung der Halogenverbindungen leitet.

In WO 97/31 882 ist ein Verfahren zur Herstellung von 1,6-Hexandiol beschrieben, bei dem ein im wesentlichen Adipinsäure und 6-Hydroxycapronsäure enthaltendes Carbonsäuregemisch, das als Nebenprodukt der Oxidation con Cyclohexanon/Cyclohexanol mit Sauerstoff oder Sauerstoff enthaltenden Gasen und durch Wasserextraktion des Reaktionsgemisches erhalten wird, mit einem niedermolukularen Alkohol zu den entsprechenden Carbonsäureestern verestert und das erhaltene Veresterungsgemisch in einer ersten Destillationsstufe von überschüssigem Alkohol und Leichtsiedern befreit, aus dem Sumpfprodukt in einer zweiten Destillationsstufe eine Auftrennung in einen von 1,4-Cyclohexandiolen im wesentlichen freie Esterfraktion und eine zumindest den größeren Teil der 1,4-Cyclohexandiole enthaltende Fraktion vorgenommen wird und die von 1,4-Cyclohexandiolen im wesentlichen freie Esterfraktion katalytisch hydriert wird.

Es hat sich nun gezeigt, daß die Aktivität des Hydrierkatalysators im Laufe der Zeit abnimmt und es wird vermutet, daß diese Desaktivierung auf den Gehalt des Edukts an organischen Halogenverbindungen zurückzuführen ist.

Es bestand daher die Aufgabe, diese organischen Halogenverbindungen vollständig oder nahezu vollständig, d.h. bis zu einem Restgehalt von unter 0,5 ppm, vorzugsweise weniger als 0,1 ppm, zu entfernen.

Die Entfernung von halogenorganischen Verunreinigung aus einem anderen Substrat ist bekannt. So wird z. B. im US 5 614 644 die Entfernung von organischen Halogenverbindungen aus Furan und hydrierten Furanen mit kupferhaltigen Katalysatoren beschrieben. Dort gelingt es jedoch nur, den Gehalt der Verunreinigung von 50 bis 2000 ppm auf weniger als 15 ppm, bevorzugt auf weniger als 5 ppm zu reduzieren.

Es wurde nun überraschenderweise gefunden, daß es bei einem Verfahren zur Herstellung von Hexandiol durch Hydrierung von Adipinsäuredialkylestern oder Gemischen, die Adipinsäuredialkylester als wesentlichen Bestandteil und die organische Halogenverbindungen als Verunreinigungen enthalten, gelingt den Gehalt an organischen Halogenverbindungen auf äußerst geringe Werte, z.B. unter 0,5 ppm, vorzugsweise unter 0,1 ppm, zu reduzieren und dadurch die Standzeit des Hydrierkatalysators stark zu erhöhen, wenn man die Adipinsäuredialkylester oder die Adipinsäuredialkylester enthaltenden Gemische vor der Hydrierung zur Entfernung der organischen Halogenverbindungen vorzugsweise in der flüssigen Phase bei einer Temperatur von 50 bis 250°C und einem Druck von 1 bis 100 bar über Kupferkatalysatoren leitet, die einem Kupfergehalt, berechnet als CuO von 0,5 bis 80 Gew.-%, eine Oberfläche von 5 bis 1500 $m^2$/g, eine Porosität von 0,05 bis 1,5 $cm^3$/g und eine Kupferoberfläche von 0,1 bis 20 $m^2$/g (jeweils pro g Katalysator) aufweisen.

Mit den erfindungsgemäßen zu verwendenden Kupferkatalysatoren gelingt nicht nur die nahezu vollständige Entfernung der Halogenverbindungen sondern sie zeichnen sich ferner dadurch aus, daß sie gegenüber dem Estergemisch chemisch stabil sind, d.h., daß in dem von den halogenhaltigen Verunreinigungen befreiten Estergemisch keine Katalysatorbestandteile nachgewiesen werden können, und daß sie das Estergemisch abgesehen von der Entfernung der halogenhaltigen Verunreinigungen in seiner Zusammensetzung chemisch nicht verändern.

Es wird angenommen, daß bei der Behandlung des Eduktstroms mit den Kupferkatalysatoren eine Spaltung der organischen Halogenverbindungen eintritt und die Kupferkatalysatoren gleichzeitig als Absorbens für die freigesetzten Halogene dienen. Deshalb werden im folgende die Begriffe Katalysator und Absorbens synonym verwendet.

Als kupferhaltige Halogenabsorbentien können sowohl Kupfer-Vollkatalysatoren als auch Kupfer-Trägerkatalysatoren verwendet werden.

Bevorzugt werden zum einen Kupfer-Trägerkatalysatoren eingesetzt, bei denen die Kupferkomponente hochdisper auf einem inerten Träger vorliegt. Als Träger eignen sich beispielsweise Aktivkohlen, Siliciumcarbid, Aluminiumoxid, Siliciumdioxid, Titandioxid, Zirconiumdioxid, Zinkoxid, Magnesiumoxid, Calciumoxid, Bariumsulfat oder deren Gemische, bevorzugt Aktivkohlen und Zirconiumdioxid. Die Träger können beispielsweise als Extrudate, Pellets, Tabletten oder Granulat eingesetzt werden.

Bevorzugt werden zum anderen Kupfer-Vollkatalysatoren eingesetzt, die neben Kupfer als weitere Bestandteile z.B. $TiO_2$, $Al_2O_3$, $ZrO_2$ oder Gemische aus diesen Verbindungen, davon bevorzugt $TiO_2$ enthalten.

Die erfindungsgemäß zu verwendenden Katalysatoren besitzen einen Kupfergehalt berechnet als CuO von 0,5 - 80 Gew.-%, bevorzugt 2-60 Gew.-%, eine Oberfläche von 5-1500 $m^2$/g, bevorzugt 10-1000 $m^2$/g, und eine Porosität von 0,05-1,5 $cm^3$/g, bevorzugt 0,1-0,8 $cm^3$/g.

Als Trägerkatalysatoren werden besonders bevorzugt zum einen $ZrO_2$-geträgte Kupferkatalysatoren verwendet, die einen Kupfergehalt berechnet als CuO von 2-8 Gew.-%, eine Oberfläche von 50-150 $m^2$/g, eine Porosität von 0,2-0,4 $cm^3$/g, und eine Kupferoberfläche von 1,0-3,0 $m^2$/g aufweisen und zum anderen Aktivkohle-geträgte Kupferkatalysatoren, die einen Kupfergehalt berechnet als CuO von 2-8 Gew.-%, eine Oberfläche von 500-1000 $m^2$/

g, eine Porosität von 0,5-0,8 cm$^3$/g, und eine Kupferoberfläche von 1,0-5,0 m$^2$/g aufweisen.

Als Vollkatalysatoren verwendet man besonders bevorzugt Kupfer-Vollkatalysatoren, die als weiterer Bestandteil neben Kupfer TiO$_2$ enthalten, die einen Kupfergehalt berechnet als CuO von 20-60 Gew.-%, eine Oberfläche von 10-150 m$^2$/g, eine Porosität von 0,1-0,5 cm$^3$/g und eine spezifische Kupferoberfläche von 0,5-3,0 m$^2$/g aufweisen.

Die verfahrensgemäß zu verwendenden Katalysatoren zeichnen sich vor allem durch eine hohe Kupferoberfläche von 0,1-20,0 m$^2$/g, bevorzugt 0,5-10,0 m$^2$/g, besonders bevorzugt 1,0-5,0 m$^2$/g Katalysator aus. Eine hohe Kupferoberfläche ist wichtig für eine effiziente Entfernung der halogenhaltigen Verunreinigungen aus dem Estergemisch und für eine hohe Aufnahmekapazität des kupferhaltigen Absorbens für die abgespaltenen Halogene. Die anspruchsgemäß festgelegte spezifische Kupferoberfläche wird mit Hilfe der im folgenden näher erläuterten N$_2$O-Puls-Chemisorption bestimmt.

### Gerät:

PulseChemiSorb 2705 der Firma Micromeritics.

### Probenvorbehandlung:

Etwa 0,3 g reduzierter Katalysator werden in einem Quarz-U-Rohrreaktor mit verbreitertem Probenteil (d$_a$ - 11mm) gegeben. Die Probe wird im Strom von 5 %H$_2$/Ar (30 ml/min) mit einer Aufheizrate von 5 K/min bis 240 °C aufgeheizt. Es folgt eine zweistündige Reduktion mit Wasserstoff bei 240°C. Die Probe wird danach 30 min in einem Helium-Strom mit 30 ml/min eluiert und unter diesem Gas auf 70°C abgekühlt.

### Durchführung der Messung:

Zur Messung der reaktiven N$_2$O-Chemisorption werden mittels einer Dosierschleife (Volumen 1000μl) N$_2$O-Pulse in einem Heliumstrom von 30 ml/min dosiert und bei 70°C durch die Probe geleitet. Es wird solange gepulst, bis vier gleiche Pulse (jeweils gleiche N$_2$O-Menge) hintereinander festgestellt werden. Die Analyse der verbrauchten Menge an N$_2$O bzw. des entstandenen N$_2$ erfolgt an einer dem Reaktor nachgeschalteten kurzen chromatographischen Trennsäule mit Porapale-N® mittels eines Wärmeleitfähigkeitsdetektors.

### Auswertung der Messung:

1. Aus der Konstanz von vier Pulsen mit dem jeweiligen Flächeninhalt PF$^i$ wird ein Mittelwert der Pulsfläche (MPF) in willkürlichen Einheiten [w.E.] ermittelt.
Bei insgesamt n Pulsen also

$$(1) \quad MPF = \sum_{i=n-3}^{n} PF^i / 4$$

Aus diesem Wert MPF können mit Hilfe des Referenzvolumens RV die Pulsflächen in μl umgerechnet werden.

$$(2) \quad xMPF[w.E.] = yRV[\mu l]$$

2. Die Menge an adsorbiertem Gas AdG wird aus dem Flächeninhalt der n Einzelpulse PF$^i$ wie folgt berechnet:

$$(3) \quad AdG[w.E.] = n*MPF - \sum_{i=1}^{n} PF^i = (n-4)*MPF - \sum_{i=1}^{n-4} PF^i$$

3. Mit Hilfe von (2) wird die Menge an adsorbiertem Gas AdG in μl berechnet.

4. Die absorbierte Gasmenge AdG [μl] wird auf Standardbedingungen AdG$^s$ [μl] umgerechnet.

$$(4) \qquad AdG^s = AdG * \frac{273*p^m}{760*T^m}$$

$p^m$ : Druck [Torr]; $T^m$ : Adsorbtionstemperatur [K]
$p^m$ und $T^m$ werden vor der Messung als Parameter eingegeben.

5. Aus AdG$^s$ wird durch die Normierung auf das Probengewicht G die spezifische adsorbierte Gasmenge $V_m$ [cm$^3$/g] berechnet.

$$(5) \qquad V_m[cm^3/g] = \frac{AdG^{s\,[\mu l]}}{1000*G[g]}$$

6. Berechnung von $V_m$ in [μmol/g]

$$(6) \qquad V_m[\mu mol/g]=44{,}940*V_m[cm^3/g]$$

7. Berechnung der spezifischen Kupferoberfläche $S^m$ [m$^2$/g$^{Kat}$] :

$$(7) \qquad S^m[m^2/g^{Kat}]= \frac{V_m[\mu mol/g]*S*N_1*10^{-6}}{K}$$

S: Stöchometriefaktor (angenommen Cu = 2)
K: Anzahl der Metallatome pro m$^2$ (für Cu = $1{,}46 \times 10^{19}$ m$^2$)
$N_1$: Avogadrozahl ($6{,}022052 \times 10^{23}$ mol$^{-1}$)

Die Herstellung der erfindungsgemäßen zu verwendenden TrägerKatalysatoren erfolgt vorzugsweise mit Hilfe des Verfahrens der Porentränkung. Dabei wird eine Kupferverbindung in einer dem Porenvolumen des Trägers entsprechenden Lösungsmittelmenge gelöst, der Träger mit der Lösung getränkt und der getränkte Katalysator anschließend 1 bis 48 h, bevorzugt 12 bis 24 h, bei Temperaturen von 80 bis 170°C, bevorzugt 100 bis 150°C getrocknet. Als Lösungsmittel wird bevorzugt Wasser verwendet. Alternativ zur Porentränkung kann auch mit einer Lösungsmenge getränkt werden, die größer ist als das Porenvolumen des Trägers (Tränkung mit überstehender Lösung). Zusätzlich kann der getrocknete Katalysator 0,5 bis 10 h, bevorzugt 1 bis 3 h, in der Regel im Luftstrom bei einer Temperatur von 200 bis 600°C, bevorzugt 250-400°C, kalziniert werden.

Als Kupferverbindungen eignen sich beispielsweise Kupfernitrat, Kupferhalogenide, Kupfercarbonat, Kupfercarboxylate, Kupferacetylacetonat oder Kupferaminkomplexe. Bevorzugt wird für die Katalysatorherstellung eine ammonikalkalische Kupfercarbonatlösung verwendet, die durch Auflösen von Kupfercarbonat in wässriger Ammoniaklösung hergestellt wird.

Die Herstellung der erfindungsgemäß zu verwendenden Vollkatalysatoren erfolgt z.B. durch Fällung einer Kupfersalzlösung in Gegenwart der anderen Katalysatorkomponenten, bevorzugt $TiO_2$, $Al_2O_3$, $ZrO_2$ oder deren Gemische, wobei die Nebenkomponenten in einer bevorzugten Ausführungsform als Pulver in einer wäßrigen Supension vorliegen. Das Kupfer wird aus der Kupfersalzlösung in an sich bekannter Weise, bevorzugt mit Sodalösung gefällt.

Die ausgefällten Niederschläge werden filtriert, alkalifrei gewaschen und bei Temperaturen von 50°C bis 150°C, bevorzugt bei 120°C getrocknet und im Anschluß gegebenenfalls bei im allgemein 200 bis 400°c, insbesondere bei 200 bis 220°C kalziniert.

Als Ausganssubstanzen können prinzipiell alle löslichen Cu(I) und/oder Cu(II)-Salze, beispielsweise Sulfate, Nitrate, Chloride, Carbonate, Acetate, Oxalate oder Ammonium-Komplexe, verwendet werden. Besonders bevorzugt wird Kupfernitrat eingesetzt.

Das wie oben beschrieben erhaltene getrocknete Pulver wird bevorzugt zu Tabletten oder ähnlichen Formkörpern verformt. Als Tablettierungshilfsmittel wird für den Verformungsprozeß Graphit, vorzugsweise in einem Anteil von 3 Gew.-%, bezogen auf das Gewicht des getrockneten Pulvers, zugegeben. Als wieteres Additiv wird zur Herstellung des Katalysators, zusätzlich zum oben beschriebenen Pulver und zu Graphit gegebenenfalls metallisches Cu-Pulver zugesetzt. Bevorzugt werden, bezogen auf das Gewicht des oben beschriebenen getrockneten Pulvers, 5 bis 40 Gew.-% metallisches Cu-Pulver zugesetzt, insbesondere 15 bis 20 Gew.-%.

Die Tablettenformkörper werden, vorzugsweise 2 Stunden, bei 300 bis 600°C, insbesondere bei 330 bis 350°C getempert. Dieses Verfahren zur Tablettierung erlaubt, im Vergleich zum ausschließlichen Einsatz von Graphit als Tablettierungshilfsmittel in den üblichen Verfahren, eine besonders leicht druchzuführende Verformung des Pulvers zu Tabletten und liefert chemisch und mechanisch sehr stabile Katalysatoren.

Vor dem Einsatz als Katalysator/Halogenabsorber müssen die Kupferkatalysatoren noch reduziert werden, um die Kupferkomponente in den metallischen Zustand zu überführen. Die Reduktion wird durchgeführt, indem man z.B. über den bereits im Reaktor befindlichen Kupferkatalysator ein Wasserstoff-enthaltendes Gas, bevorzugt $H_2/N_2$-Gemische mit $H_2$-Gehalten von 50-100 Vol.-%, bei einer Temperatur von 100-250°C, bevorzugt 150-200°C, über einen Zeitraum von 10-48 h, bevorzugt 20-30 h, leitet.

Die Entfernung der halogenhaltigen Verunreinigungen aus dem Estergemisch mit den kupferhaltigen Katalysatoren/Halogenabsorbentien kann sowohl in der Flüssigphase als auch in der Gasphase durchgeführt werden. Bevorzugt wird in der Flüssigphase gearbeitet. Die Entfernung der halogenhaltigen Verunreinigungen aus dem Estergemisch kann diskontinuierlich oder kontinuierlich durchgeführt werden. Bevorzugt wird im kontinuierlichen Betrieb gearbeitet.

Bei der Entfernung der halogenhaltigen Verunreinigung in der Flüssigphase im kontinuierlichen Betrieb wird das kupferhaltige Halogenabsorbens beispielsweise in einen Rohrreaktor eingefüllt, im Rohrreaktor wie oben beschrieben durch Behandlung mit einem Wasserstoff-enthaltenden Gas aktiviert, und das die halogenhaltigen Verunreinigungen enthaltende Estergemisch bei einer Flüssigkeitsbelastung von 0,05-50 kg Estergemsich/l Katalysator * h, einer Temperatur von 50-250°C, bevorzugt 100-200°C, besonders bevorzugt 150-180°C, und einem Druck, der so hoch gewählt wird, daß sich keine Gasphase ausbilden kann, beispielsweise 1-100 bar, bevorzugt 1-10 bar, besonders bevorzugt 1-5 bar, entweder von unten nach oben (Sumpffahrweise) oder von oben nach unten (Rieselfahrweise) über den Katalysator geleitet. Bevorzugt wird in Sumpffahrweise gearbeitet. Der Konzentration der halogenhaltigen Verbindungen im gereinigten Estergemisch liegt im allgemeinen unter 0,5 ppm, bevorzugt unter 0,1 ppm Cl.

Als erfindungsgemäß zu reinigende Adipinsäuredialkylester, insbesondere Ester der Adipinsäure mit niedermolekularen Alkoholen z.B. mit Alkoholen mit 1 bis 4 C-Atomen, oder diese enthaltende Estergemische kommen Edukte beliebiger Herkunft in Betracht, die aufgrund ihrer Herstellungsmethode organische Halogenverbindungen als Verunreinigungen enthalten.

Bevorzugt verwendet man jedoch als Edukt eine Esterfraktion, wie sie erhalten wird

a) durch Veresterung eines wässrigen Dicarbonsäuregemisches, das Adipinsäure, 6-Hydroxycapronsäure und geringe Mengen 1,4-Cyclohexandiole enthält und das als Nebenprodukt bei der Oxidation von Cyclohexan zu Cyclohexanon/Cyclohexanol mit Sauerstoff oder Sauerstoff enthaltenden Gasen und durch Wasserextraktion des Reaktorgemisches anfällt, mit einem niedermolekularen Alkohol, bevorzugt n- oder i-Butanol und insbesondere Methanol,

b) Entfernung des überschüssigen Alkohols und der Leichtsieder in einer ersten Destillationsstufe und

c) Auftrennung des Sumpfproduktes in einer zweiten Destillationsstufe in eine zumindest den grösseren Teil der cyclohexandiole enthaltende Fraktion und eine von 1,4-Cyclohexandiolen im wesentlichen freien und zu hydrierende Esterfraktion.

Die Herstellung dieser Esterfraktion ist in WO 97/31882 eingehend beschrieben. Sie enthält in Konzentrationen von bis zu 1000 ppm, in der Regel bis zu 100 ppm, und besonders weniger als 10 ppm halogenhaltige organische Verbindungen, wie chlorierte Cyclohexane, vor allem Monochlorcyclohexan, 1,2-Dichlorcyclohexan, 1,3-Dichlorcyclohexan sowie 1,4-Dichlorcyclohexan als Verunreinigungen, die destillativ nicht abgetrennt werden können.

Diese organischen Halogenverbindungen werden an den erfindungsgemäß zu verwendenden Kupferkatalysatoren auf in der Regel weniger als 0,1 ppm häufig unter die Nachweisgrenze gespalten und absorbiert. Die Hydrierung der von den organischen Halogenverbindungen befreiten Adipinsäuredialkylester oder der diese enthaltende Estergemische erfolgt in an sich bekannter Weise und mit an sich bekannten Katalysatoren. Hydrierungsbedingungen und Katalysatoren sind im einzelnen in WO 97/31882 beschrieben, so daß auf diese Schrift ausdrücklich Bezug genommen wird und sie als hier inkorporiert gelten soll. die Merkmale des in WO 97/31882 beschriebenen Verfahrens sind dabei im wesentlichen folgende:

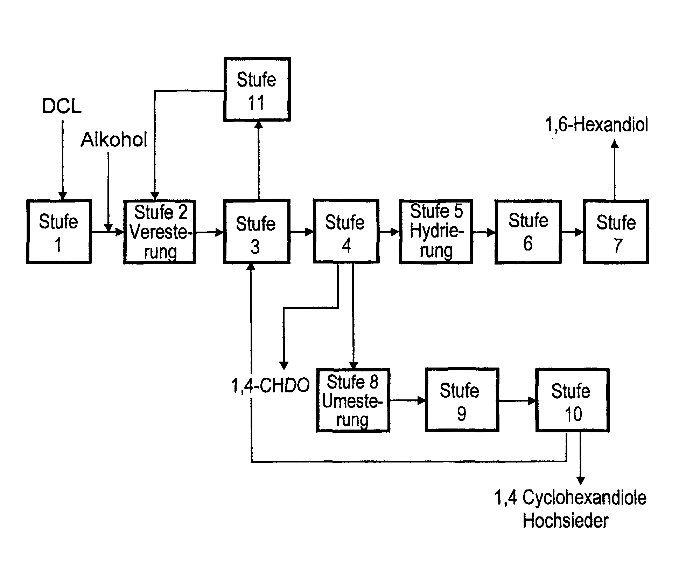

Zum besseren Verständnis wird das Verfahren zur Herstellung von Hexandiol gemäß Fig. 1 erläutert, in der die einzelnen Verfahrensschritte in weitere Stufen aufgeschlüsselt sind, wobei die Stufen 2, 3, 4, 5, 6, 7 für das Verfahren essentiell sind und die Stufen 3 und 4 sowie 6 und 7 auch zusammengefaßt werden können. Die Stufen 8, 9, 10 und 11 sind fakultativ, aber zur Erhöhung der Wirtschaftlichkeit des Verfahrens gegebenenfalls sinnvoll.

Die Dicarbonsäurelösung (DCL) ist im allgemeinen eine wäßrige Lösung mit einem Wasseranteil von 20 bis 80 %. Da eine Veresterungsreaktion eine Gleichgewichtsreaktion darstellt, ist es meist sinnvoll, insbesondere bei Veresterung mit z.B. Methanol, vorhandenes Wasser vor der Reaktion zu entfernen, vor allem, wenn während der Veresterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders bevorzugt 30 bis 200°C und einem Druck von 1 bis 1500 mbar, bevorzugt 5 bis 1100 mbar, besonders bevorzugt 20 bis 1000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, daß das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.% betragen.

Die Abtrennung des Wassers kann so erfolgen, daß das Wasser überwiegend säurefrei erhalten wird, oder man kann die in der DCL enthaltenen niederen Monocarbonsäuren - im wesentlichen Ameisensäure - zum größten Teil mit dem Wasser abdestillieren, damit diese in der Veresterung keinen Veresterungsalkohol binden.

Dem Carbonsäurestrom aus der Stufe 1 wird ein Alkohol mit 1 bis 10 C-Atomen zugemischt, gemäß Variante A Alkohole mit 1 bis 3 Kohlenstoffatomen, d.s. Methanol, Ethanol, Propanol oder iso-Propanol, bevorzugt Methanol, gemäß Variante B Alkohole mit 4 bis 10, insbesondere 4 bis 8 Kohlenstoffatomen und besonders bevorzugt n-Butanol, iso-Butanol, n-Pentanol und i-Pentanol.

Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400°C, bevorzugt bei 70 bis 300°C, besonders bevorzugt bei 90 bis 200°C durchgeführt werden. Es kann ein äußerer Druck angelegt werden, bevorzugt wird die Veresterung aber unter Eigendruck des Reaktionssystems durchgeführt. Als Veresterungsapparate können dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen; bevorzugt wird aber zur Erhöhung der Reaktionsgeschwindigkeit ein Katalysator zugesetzt. Dabei kann es sich um einen homogen gelösten oder um einen festen Katalysator handeln. Als homogene Katalysatoren seien beispielhaft Schwefelsäure, Phosphorsäure, Salzsäure, Sulfonsäuren, wie p-Toluolsulfonsäure, Heteropolysäuren wie Wolframatophosphorsäure oder Lewissäuren wie Aluminium-, Vanadium-, Titan-, Bor-Verbindungen genannt. Bevorzugt sind Mineralsäuren, insbesondere Schwefelsäure. Das Gew.-Verhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie $SiO_2$, $Al_2O_3$, $SnO_2$, $ZrO_2$ oder Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt, abzüglich der gegebenenfalls zugesetzten Säure als Katalysator, 0,01 bis 50, bevorzugt 0,1 bis 10. Dabei liegen nicht alle im System vorhandenen Carboxylgruppen als Ester des eingesetzten Alkohols vor, sondern ein Teil kann in Form von dimeren oder oligomeren Estern, z.B. mit dem OH-Ende der Hydroxycapronsäure vorliegen.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1500 mbar, bevorzugt 20 bis 1000 mbar, besonders bevorzugt 40 bis 800 mbar und Temperaturen zwischen 0 und 150°C, bevorzugt 15 und 90°C, und insbesondere 25 und 75°C der überschüssige Veresterungsalkohol ROH, Wasser sowie z.B. entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren, wie Adipinsäure und Glutarsäure, Hydroxycarbonsäuren, wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 10 Gew.-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf im allgemeinen zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drucken von 1 bis 1000 mbar, bevorzugt 5 bis 500 mbar, besonders bevorzugt 10 bis 200 mbar, betrieben.

Nach Variante A, d.h. der veresterung mit $C_1$- bis $C_3$-Alkoholen, insbesondere Methanol, wird nun der Strom aus Stufe 3 in eine zu hydrierende Kopffraktion und eine die 1,4-Cyclohexandiole enthaltende Sumpffraktion getrennt.

Die Kopffraktion besteht überwiegend aus Restwasser und Restalkohol ROH, Estern des Alkohols ROH mit Monocarbonsäuren, überwiegend $C_3$- bis $C_6$-Monocarbonsäuren, Estern mit Hydroxycarbonsäuren, wie 6-Hydroxycapronsäure, 5-Hydroxyvaleriansäure, sowie vor allem den Diestern mit Dicarbonsäuren, wie Adipinsäure, Glutarsäure und Bernsteinsäure, ferner 1,2-Cyclohexandiolen, Caprolacton und Valerolacton.

Die genannten Komponenten können zusammen über Kopf abgetrennt und in die Hydrierung (Stufe 5) eingeschleust werden oder in einer weiteren bevorzugten Ausführungsform in der Kolonne in einen Kopfstrom, der überwiegend Restwasser und Restalkohol sowie die oben erwähnten Ester der $C_3$- bis $C_5$-Carbonsäuren enthält und einen Seitenstrom, der überwiegend die oben erwähnten Ester der $C_6$-Carbonsäuren und Dicarbonsäuren enthält, die dann in die Hydrierung gelangen, aufgetrennt werden.

Die schwersiedenden Komponenten des Stromes aus Stufe 4, überwiegend bestehend aus 1,4-Cyclohexandiolen oder deren Ester, dimeren oder oligomeren Estern sowie nicht näher definierten z.T. polymeren Bestandteilen der DCL, werden über den Abtriebsteil der Kolonne abgetrennt. Diese können zusammen anfallen oder so, daß über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Die schwersiedenden Komponenten, mit oder ohne den Gehalt an 1,4-Cyclodiolen, können entweder verbrannt werden oder in einer bevorzugten Ausführungsform zur sog. Umesterung in die Stufe 8 gelangen.

Nach Variante B, d.h. der Veresterung mit $C_4$- bis $C_{10}$-Alkoholen, insbesondere n- oder i-Butanol, kann der Strom aus Stufe 3 in der Stufe 4 in eine die 1,4-Cyclohexandiole enthaltene Kopffraktion, einen vorwiegend die $C_6$-Ester enthaltenden Seitenstrom, der in die Hydrierung gelangt und Hochsieder enthaltenden Sumpfstrom, der gegebenenfalls in die Stufe 8 gelangen kann, aufgetrennt werden.

Die Kopffraktion besteht überwiegend aus Restalkohol ROH, $C_1$- bis $C_3$-Monoestern des Alkohols ROH, Valerolacton und 1,2- und 1,4-Cyclohexandiolen.

Der Seitenstrom enthält überwiegend Diester von Bernsteinsäure, Glutarsäure und Adipinsäure sowie Monoester der 5-Hydroxyvaleriansäure und 6-Hydroxycapronsäure. Dieser Seitenstrom kann entweder oberhalb oder auch unterhalb der Zulaufstelle der Kolonne entnommen werden und in die Hydrierung (Stufe 5) eingeschleust werden.

Der Sumpfstrom mit oligomeren Estern und sonstigen Hochsiedern kann entweder verbrannt oder vorteilhaft in die Stufe 8 gelangen.

Nach einer weiteren Ausführungsform werden in der Stufe 4 die $C_6$-Ester zusammen mit entweder dem Sumpfstrom abgetrennt und dann, in einer weiteren Kolonne, entweder als Sumpfprodukt von der bereits beschriebenen Kopffraktion, die überwiegend aus Restalkohol ROH, $C_1$- bis $C_3$-Monoestern des Alkohols ROH, Valerolacton und 1,2- und 1,4-Cyclohexandiolen besteht, oder als Kopfstrom von den Hochsiedern abgetrennt.

Die von 1,4-Cyclohexandiole freie oder praktisch freie Fraktion der Stufe 4, entweder der Gesamtstrom oder der hauptsächlich Ester der $C_6$-Säuren enthaltende Seitenstrom, wird in die Hydrierstufe 5 geleitet.

Die Stufen 3 und 4 können, insbesondere wenn nur kleinere Mengen verarbeitet werden, zusammengefaßt werden. Dazu kann beispielsweise in einer absatzweise durchgeführten fraktionierten Destillation der $C_6$-Esterstrom gewonnen werden, wiederum ohne daß 1,4-Cyclohexandiole in den zur Hydrierung geführten Strom gelangen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind z.B. in Houben-Weyl, Methoden der Organischen Chemie, Band IV/1c, Georg Thieme Verlag Stuttgart, 1980, S. 45- 67) und Beispiele für heterogene Katalysatoren sind z.B. in Houben-Weyl, Methoden der Organischen Chemie, Band IV/1c, S. 16 bis 26 beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. $Cr_2O_3$, $Al_2O_3$, $SiO_2$, $ZrO_2$, $ZnO_2$, BaO und MgO oder Mischungen daraus.

Besonders bevorzugt sind Katalysatoren, wie sie in EP 0 552 463 beschrieben sind. Dies sind Katalysatoren, die in der oxidischen Form die Zusammensetzung

$$Cu_aAl_bZr_cMn_dO_x$$

besitzen, wobei a > 0, b > 0, c > 0, d > 0, a > b/2, b> a/4, a > c und a > d gilt und x die zur Wahrung der Elektroneutralität pro Formeleinheit erforderlichen Anzahl von Sauerstoffionen bezeichnet. Die Herstellung dieser Katalysatoren kann beispielsweise nach den Angaben der EP 552 463 durch Fällung von schwerlöslichen Verbindungen aus Lösungen erfolgen, welche die entsprechenden Metallionen in Form ihrer Salze enthalten.

Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so daß man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfläche der Katalysatoren liegt zwischen 10 und 150 m$^2$/g.

Bevorzugt werden Heterogenkatalysatoren verwendet, die entweder fest angeordnet oder als Supension eingesetzt werden. Wird die Hydrierung in der Gasphase und über fest angeordnetem Katalysator durchgeführt, werden im allgemeinen Temperaturen von 150 bis 300°C bei Drücken von 1 bis 100 bar, bevorzugt 15 bis 70 bar angewandt. Dabei wird mindestens so viel Wasserstoff als Hydriermittel und Trägergas verwendet, daß Edukte. Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z.B. Methan ausgeschleust werden kann. Es können dabei ein Reaktor oder mehrere Reaktoren hintereinander geschaltet verwendet werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinandergeschalteten Reaktoren durchgeführt werden. Die Hydrierung in Flüssigphase über ein Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann diskontinuierlich, bevorzugt kontinuierlich erfolgen.

Der Hydrieraustrag besteht im wesentlichen aus 1,6-Hexandiol und dem Alkohol ROH. Weitere Bestandteile sind, vor allem falls der gesamte leichtsiedende Strom der Stufe 4 gemäß Variante A eingesetzt wurde, 1,5-Pentandiol, 1,4-Butandiol, 1,2-Cyclohexandiole sowie kleine Mengen von Monoalkoholen mit 1 bis 6 C-Atomen und Wasser.

Dieser Hydrieraustrag wird in der Stufe 6, die z.B. ein Membransystem oder bevorzugt eine Destillationskolonne ist, in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1500 mbar, bevorzugt 30 bis 1200 mbar, besonders bevorzugt 50 bis 1000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 2 zurückgeführt werden oder in die Stufe 8 oder in die Stufe 11 gelangen.

Der 1,6-Hexandiol enthaltene Stoffstrom wird in der Stufe 7 in einer Kolonne gereinigt. Dabei werden 1,5-Pentandiol, gegebenenfalls die 1,2-Cyclohexandiole, sowie weitere eventuell vorhandene Leichtsieder, über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99 % aus einem Seitenstrom der Kolonne entnommen. Dabei werden bei Drücken von 1 bis 1000 mbar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Das erfindungsgemäße Verfahren wird anhand der nachfolgenden Beispiele näher erläutert, aber dadurch in keiner Weise eingeschränkt.

**Herstellung Katalysator A:**

Ein Aktivkohle-Träger in Form von 2-5 mm-Granulat wird mit einer ammoniakalkalischen Kupfercarbonatlösung, die durch Auflösen von Kupfercarbonat in einer 25 Gew.-%igen wässrigen Ammoniaklösung hergestellt wurde, getränkt, wobei das Volumen der Tränklösung dem Porenvolumen der eingesetzten Trägermenge entspricht. Der getränkte Trager wird 16 h bei 120°C getrocknet. Der so hergestellte Katalysator besitzt einen Kupfergehalt, berechnet als CuO von 6,0 Gew.-%, eine Oberfläche von 820 m$^2$/g, eine Porosität von 0,65 cm$^3$/g und nach Reduktion eine Kupferoberfläche von 2,9 m$^2$/g.

**Herstellung Katalysator B:**

Ein Zirconiumdioxid-Träger in Form von 3 mm-Extrudaten wird mit einer ammoniakalkalischen Kupfercarbonatlösung, die durch Auflösen von Kupfercarbonat in einer 25 Gew.-%igen wässrigen Ammoniaklösung hergestellt wurde, getränkt, wobei das Volumen der Tränklösung dem Porenvolumen der eingesetzten Trägermenge entspricht. Der getränkte Träger wird 16 h bei 120°C getrocknet und anschließen 1 h bei 350°C kalziniert. Der so hergestellte Katalysator besitzt einen Kupfergehalt berechnet als CuO von 5,0 Gew.-%, eine Oberfläche von 74 m$^2$/g, eine Porosität von 0,30 cm$^3$/g und nach Reduktion eine Kupferoberfläche von 1,1 m$^2$/g.

**Herstellung Katalysator C:**

Katalysator C wurde durch Fällung einer Lösung von Kupfernitrat mit Sodalösung hergestellt. Als Vorlage wurde eine Suspension von TiO$_2$ in Wasser verwendet. Das bei der Fällung entstehende Fällgut wurde abfiltriert, gewaschen und bei 120°C getrocknet. Das getrocknete Pulver wurde zwei Stunden bei 200°C calciniert und danach mit 3 % Graphit und 20 % metallischen Kupferpulver zu Tabletten mit 3 mm verpreßt. diese Tabletten wurden 2 Stunden bei 330°c getempert.

Der so hergestellte Katalysator besitzt einen Kupfergehalt, berechnet als CuO, von 44,0 Gew.-%, eine Oberfläche von 39 m$^2$/g, eine Porosität von 0,22 cm$^3$/g und nach Reduktion eine Kupferoberfläche von 1,4 cm$^3$/g.

Beispiel 1

a) Entfernung der Cl-haltigen Verunreinigungen (Stufen 1 bis 4 gemäß Beispiel WO 97/31882)

Stufe 1 (Entwässerung)

0,1 kg/h Dicarbonsäurelösung (bestehend im wesentlichen aus Adipinsäure, 6-Hydroxycapronsäure, 1,4-Cyclohexandiolen, Glutarsäure, 5-Hydroxyvaleriansäure, Ameisensäure, Wasser) wurden kontinuierlich in eine Destillationsapparatur (dreibödige Glockenbodenkolonne mit außenligendem Öl-Heizkreislauf, Öltemperatur 150°c, Bodenvolumen je ca. 25 ml, Zulauf über den Glockenboden) mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (5,5 kg) betrug der Wassergehalt ca. 0,4 %.

Stufe 2 (Veresterung):

5,5 kg/h des Sumpfstroms aus Stufe 1 wurden mit 8,3 kg/h Methanol und 14 g/h Schwefelsäure kontinuierlich in einem Rohrreaktor (1 0,7 m, ∅ 1,8 cm, Verweilzeit 2,7 h) umgesetzt. Die Säurezahl des Austrags abzüglich Schwefelsäure betrug ca. 10 mg KOH/g.

Stufe 3 (Entfernen überschüssigen Alkohols und von Wasser):

In einer 20 cm Füllkörperkolonne wurde der Veresterungsstrom aus Stufe 2 destilliert (1015 mbar, 65°C Kopftemperatur, bis 125°C Sumpftemperatur). Über Kopf wurden 7,0 kg abgezogen. Als Sumpfprodukte wurden 6,8 kg erhalten.

Stufe 4 (Fraktionierung; 1,4-Cyclohexandiolabtrennung):

In einer 50 cm Füllkörperkolonne wurde der Sumpfstrom aus Stufe 3 fraktioniert destilliert (1 mbar, 70-90°C Kopftemperatur, bis 180°C Sumpftemperatur). Der Sumpf (1,9 kg) enthielt praktisch alle 1,4-Cyclohexandiole.

Als Leichtsieder wurden 0,6 kg abdestilliert (1,2-Cyclohexandiole, Valerolacton, 5-Hydroxyvaleriansäuremethylester, Glutarsäuredimethylester, Bernsteinsäuredimethylester u.a.). Als überwiegend Adipinsäuredimethylester und 6-Hydroxycapronsäuremethylester enthaltende Fraktion wurden 4,3 kg erhalten.

Ein Rohrreaktor wurde mit 50 ml Katalysator A befüllt. Der Katalysator wurde zunächst reduziert, indem über den im Reaktor befindlichen Katalysator über einen Zeitraum von 24 h reiner Wasserstoff bei einer Temperatur von 180°C geleitet wurde. Anschließend wurde die überwiegend Adipinsäuremethylester und Hydroxycapronsäuremethylester enthaltende Fraktion gemäß Stufe 4, die einen Cl-Gehalt von 0,7 ppm Cl aufwies, bei einer Flüssigkeitsbelastung von 0,5 kg Estergemisch/l Katalysator * h, einer Temperatur von 170°C und einem Druck von 1 bar von unten nach oben (Sumpffahrweise) über den Katalysator geleitet. Der Cl-Gehalt des Reaktoraustrags lag unterhalb der Nachweisgrenze von 0,1 ppm. Im Reaktoraustrag konnten keine Katalysatorbestandteile nachgewiesen werden, d.h. der Katalysator ist chemisch gegenüber dem Estergemisch absolut stabil. GC-analytisch konnte keine Veränderung der Zusammensetzung des Estergemisches vor und nach der Entfernung der Cl-haltigen Verunreinigungen festgestellt werden.

b) Hydrierung (Stufe 5)

27 kg des von den Cl-haltigen Verunreinigungen gereinigten $C_6$-Estergemisches aus Stufe a) wurden kontinuierlich in einem 25 ml Reaktor an einem Katalysator hydriert (Katalysator: 70 Gew.-%, CuO, 25 Gew.-% ZnO, 5 Gew.-% $Al_2O_3$), der zuvor im Wasserstoffstrom bei 180°C aktiviert worden ist (Hydrierbedingungen: Zulauf 20 g/h, kein Umlauf, 220 bar, 220°C). Der Ester-Umsatz lag über die gesamte Versuchsdauer bei 99,5 %, die 1,6-Hexandiolselektivität betrug 99%.

Beispiel 2:

Beispiel 1 wurde wiederholt mit der Änderung, daß in Stufe a) der Rohrreaktor mit 50 ml Katalysator B befüllt wurde. Der Katalysator wurde zunächst reduziert, indem über den im Reaktor befindlichen Katalysator über einen Zeitraum von 24 h reiner Wasserstoff bei einer Temperatur von 180°C geleitet wurde. Anschließend wurde die überwiegend Adipinsäuremethylester und Hydroxycapronsäuremethylester enthaltende Fraktion gemäß Beispiel 1, die einen Cl-Gehalt von 0,7 ppm Cl aufwies, bei einer Flüssigkeitsbelastung von 0,5 kg Estergemisch/l Katalysator * h, einer Temperatur von 170°C und einem Druck von 1 bar von unten nach oben (Sumpffahrweise) über den Katalysator geleitet. Der Cl-Gehalt des Reaktoraustrags lag unterhalb der Nachweisgrenze von 0,1 ppm. Im Reaktoraustrag konnte keine Katalysatorbestandteile nachgewiesen werden, d.h. der Katalysator ist chemisch gegenüber dem Estergemisch absolut stabil. GC-analytisch konnte keine Veränderung der Zusammensetzung des Estergemisches vor und nach der Entfernung der Cl-haltigen Verunreinigungen festgestellt werden. Die Hydrierergebnisse waren die gleichen wie in Beispiel 1.

Vergleichsbeispiel 3:

Analog Beispiel 1 wurde das Verfahren bis einschließlich zur Hydrierung durchgeführt, aber ohne daß das halogenhaltige $C_6$-Estergemisch vor der Hydrierung über ein kupferhaltiges Halogenabsorbens geleitet wurde, d.h. ohne Stufe a). Zu Beginn der Hydrierung betrug der Ester-Umsatz 99,5 %, die Selektivität 99 %. Aber bereits nachdem 6,0 kg der insgesamt 27 kg des zu hydrierenden $C_6$-Estergemisches umgesetzt worden waren, war infolge der Desaktivierung des Hydrierkatalysators durch die halogenhaltigen Verunreinigungen der Esterumsatz auf nur noch 90 % und die Selektivität auf 97 % abgesunken.

Beispiel 4:

Beipiel 1 wurde wiederholt mit der Änderung, daß in Stufe a) der Rohrreaktor mit 50 ml Katalysator C befüllt wurde. Der Katalysator wurde zunächst reduziert, indem über den im Reaktor befindlichen Katalysator über einen Zeitraum von 24 h reiner Wasserstoff bei einer Temperatur von 180°C geleitet wurde. Anschließend wurde die überwiegend Adipinsäuremethylester und Hydroxycapronsäuremethylester enthaltende Fraktion gemäß Beispiel 1, die einen Cl-Gehalt von 0,7 ppm Cl aufwies, bei einer Flüssigkeitsbelastung von 0,5 kg Estergemisch/ 1 Katalysator * h, einer Temperatur von 150°c und einem Druck von 1 bar von unten nach oben (Sumpffahrweise) über den Katalysator geleitet. Der C1-gehalt des Reaktoraustrags lag unterhalb der Nachweisgrenze von 0,1 ppm. Im Reaktoraustrag konnte keine Katalysatorbestandteile nachgewiesen werden, d.h. der Katalysator ist chemisch gegenüber dem Estergemisch absolut stabil. GC-analytisch konnte keine Veränderung der Zusammensetzung des Estergemisches vor und nach der Entfernung der Cl-haltigen Verunreinigungen festgestellt werden. Die Hydrierergebnisse waren die gleichen wie in Beispiel 1.

**Patentansprüche**

**1.** Verfahren zur Herstellung von Hexandiol durch Hydrierung von Adipinsäuredialkylestern oder Gemischen, die Adipinsäuredialkylester als wesentlichen Bestandteil und die organische Halogenverbindungen als Verunreinigungen enthalten, **dadurch gekennzeichnet, daß** man die Adipinsäuredialkylester oder die Adipinsäuredialkylester

EP 1 082 282 B1

enthaltenden Gemische vor der Hydrierung zur Entfernung der organischen Halogenverbindungen bei einer Temperatur von 50 bis 250°C und einem Druck von 1 bis 100 bar über Kupfer-Katalysatoren leitet, die einen Kupfergehalt, berechnet als CuO von 0,5 bis 80 Gew.-%, eine Oberfläche von 5 bis 1500 $m^2/g$, eine Porosität von 0,05 bis 1,5 $cm^3/g$ und eine Kupferoberfläche von 0,1 bis 20 $m^2/g$ aufweisen.

2. Verfahren nach Anspruch 1, **dadurch gekennzeichnet, daß** man Kupfer-Katalysatoren verwendet, die einen Kupfergehalt berechnet als CuO von 2 bis 60 Gew.-%, eine Oberfläche von 10 bis 1000 $m^2/g$, eine Porosität von 0,1 bis 0,8 $cm^3/g$ und eine spezifische Kupferoberfläche von 0,5 bis 10,0 $m^2/g$ aufweisen.

3. Verfahren nach Anspruch 1, **dadurch gekennzeichnet**, damß man $TiO_2$-haltige Kupfer-Vollkatalysatoren verwendet, die einen Kupfergehalt, berechnet als CuO, von 20 bis 60 Gew.-%, eine Oberfläche von 10 bis 150 $m^2/g$, eine Porosität von 0,1 bis 0,5 $cm^3/g$ und eine spezifische Kupferoberfläche von 0,5 bis 3,0 $m^2/g$ aufweisen

4. Verfahren nach Anspruch 1, **dadurch gekennzeichnet, daß** man $ZrO_2$-geträgerte Kupferkatalysatoren verwendet, die einen Kupfergehalt, berechnet als CuO, von 2 bis 8 Gew.-%, eine Oberfläche von 50 bis 150 $m^2/g$, eine Porosität von 0,2 bis 0,4 $cm^3/g$ und eine spezifische Kupferoberfläche von 1,0 bis 3,0 $m^2/g$ aufweisen.

5. Verfahren nach Anspruch 1, **dadurch gekennzeichnet, daß** man Aktivkohle-geträgerte Kupferkatalysatoren verwendet, die einen Kupfergehalt, berechnet als CuO, von 2 bis 8 Gew.-%, eine Oberfläche von 500 bis 1000 $m^2/g$, eine Porosität von 0,5 bis 0,8 $cm^3/g$ und eine spezifische Kupferoberfläche von 1,0 bis 5,0 $m^2/g$ aufweisen.

6. Verfahren nach Anspruch 1, **dadurch gekennzeichnet, daß** die halogenhaltigen Verunreinigungen in der Flüssigphase im kontinuierlichen Betrieb bei einer Flüssigkeitsbelastung von 0,05 - 50 kg Estergemisch/1 Katalysator * h, einer Temperatur von 50 - 250°C und einem Druck von 1 - 100 bar entfernt werden.

7. Verfahren nach Anspruch 1, **dadurch gekennzeichnet, daß** die halogenhaltigen Verunreinigungen in der Flüssigphase im kontinuierlichen Betrieb bei einer Flüssigkeitsbelastung von 0,1 - 10 kg Estergemisch/1 Katalysator * h, einer Temperatur von 100 - 200°C und einem Druck von 1-10 bar entfernt werden.

8. Verfahren nach Anspruch 1, **dadurch gekennzeichnet, daß** die halogenhaltigen Verunreinigungen in der Flüssigphase im kontinuierlichen Betrieb bei einer Flüssigkeitsbelastung von 0,2 - 1,0 kg Estergemisch/1 Katalysator * h, einer Temperatur von 150 - 180°C und einem Druck von 1 - 5 bar entfernt werden.

9. Verfahren nach Anspruch 1, **dadurch gekennzeichnet, daß** man als Edukt eine Esterfraktion verwendet, wie sie erhalten wird

   (a) durch Veresterung eines wässrigen Dicarbonsäuregemisches, das Adipinsäure, 6-Hydroxycapronsäure und geringe Mengen 1,4-Cyclohexandiole enthält und das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemisches anfällt, mit einem niedermolukularen Alkohol,

   (b) Entfernung des überschüssigen Alkohols und der Leichtsieder in einer ersten Destillationsstufe und

   (c) Auftrennung des Sumpfproduktes in einer zweiten Destillationsstufe in eine zumindest den grösseren Teil der Cyclohexandiole enthaltende Fraktion und eine von 1,4-Cyclohexandiolen im wesentlichen freie und zu hydrierende Esterfraktion.

10. Verfahren nach Anspruch 1, **dadurch gekennzeichnet, daß** man den Gehalt an organischen Halogenverbindungen auf weniger als 0,1 ppm vermindert.

**Claims**

1. A process for the preparation of hexanediol by hydrogenating dialkyl adipates or mixtures which contain a dialkyl adipate as the essential component and organic compounds as impurities, wherein, before the hydrogenation, the dialkyl adipates or the mixtures containing dialkyl adipates are passed at from 50 to 250°C and from 1 to 100 bar over copper catalysts which have a copper content, calculated as CuO, of from 0.5 to 80% by weight, a surface area of from 5 to 1500 $m^2/g$, a porosity of from 0.05 to 1.5 $cm^3/g$ and a copper surface area of from 0.1 to 20 $m^2/$

g, in order to remove the organic halogen compounds.

2. A process as claimed in claim 1, wherein copper catalysts which have a copper content, calculated as CuO, of from 2 to 60% by weight, a surface area of from 10 to 1000 $m^2$/g, a porosity of from 0.1 to 0.8 $cm^3$/g and a specific copper surface area of from 0.5 to 10.0 $m^2$/g are used.

3. A process as claimed in claim 1, wherein $TiO_2$-containing unsupported copper catalysts which have a copper content, calculated as CuO, of from 20 to 60% by weight, a surface area of from 10 to 150 $m^2$/g, a porosity of from 0.1 to 0.5 $cm^3$/g and a specific copper surface area of from 0.5 to 3.0 $m^2$/g are used.

4. A process as claimed in claim 1 wherein $ZrO_2$-supported copper catalysts which have a copper content, calculated as CuO, of from 2 to 8% by weight, a surface area of from 50 to 150 $m^2$/g, a porosity of from 0.2 to 0.4 $cm^3$/g and a specific copper surface area of from 1.0 to 3.0 $m^2$/g are used.

5. A process as claimed in claim 1, wherein active carbon-supported copper catalysts which have a copper content, calculated as CuO, of from 2 to 8% by weight, a surface area of from 500 to 1000 $m^2$/g, a porosity of from 0.5 to 0.8 $cm^3$/g and a specific copper surface area of from 1.0 to 5.0 $m^2$/g are used.

6. A process as claimed in claim 1, wherein the halogen-containing impurities are removed in the liquid phase in continuous operation with a liquid space velocity of 0.05 - 50 kg of ester mixture per l of catalyst per hour at 50 - 250°C and at 1 - 100 bar.

7. A process as claimed in claim 1, wherein the halogen-containing impurities are removed in the liquid phase in continuous operation with a liquid space velocity of 0.1 - 10 kg of ester mixture per l of catalyst per hour at 100 - 200°C and at 1 - 10 bar.

8. A process as claimed in claim 1, wherein the halogen-containing impurities are removed in the liquid phase in continuous operation with a liquid space velocity of 0.2 - 1.0 kg of ester mixture per l of catalyst per hour at 150 - 180°C and at 1 - 5 bar.

9. A process as claimed in claim 1, wherein the starting material used is an ester fraction as obtained

(a) by esterification of an aqueous dicarboxylic acid mixture, which contains adipic acid, 6-hydroxycaproic acid and small amounts of 1,4-cyclohexanediols and which is obtained as a byproduct in the oxidation of cyclohexane to cyclohexanone/cyclohexanol with oxygen or oxygen-containing gases and by water extraction of the reaction mixture, with a low molecular weight alcohol,

(b) removal of the excess alcohol and of the low boilers in a first distillation stage and

(c) separation of the bottom product in a second distillation into a fraction containing at least the major part of the cyclohexanediols and an ester fraction which is essentially free of 1,4-cyclohexanediols and is to be hydrogenated.

10. A process as claimed in claim 1, wherein the content of organic halogen compounds is reduced to less than 0.1 ppm.

**Revendications**

1. Procédé pour la préparation de hexanediol au moyen d'une hydrogénation d'esters dialkyliques de l'acide adipique ou de mélanges contenant des esters dialkyliques de l'acide adipique en tant que composant principal ainsi que des composés halogénés organiques en tant qu'impuretés, **caractérisé en ce que** l'on fait passer les esters dialkyliques de l'acide adipique ou les mélanges contenant les esters dialkyliques de l'acide adipique, avant de réaliser l'hydrogénation afin d'éliminer les composés halogénés organiques, sur des catalyseurs de cuivre à une température allant de 50°C à 250°C et sous une pression allant de 1 bar à 100 bars, ces catalyseurs présentant une teneur en cuivre, exprimée en CuO, allant de 0,5% à 80% en poids, une surface spécifique allant de 5 $m^2$/g à 1 500 $m^2$/g, une porosité allant de 0,05 $cm^3$/g à 1,5 $cm^3$/g et une surface spécifique de cuivre allant de 0,1 $m^2$/g à 20 $m^2$/g.

**2.** Procédé selon la revendication 1, **caractérisé en ce que** l'on met en oeuvre des catalyseurs de cuivre présentant une teneur en cuivre, exprimée en CuO, allant de 2% à 60% en poids, une surface spécifique allant de 10 $m^2/g$ à 1 000 $m^2/g$, une porosité allant de 0,1 $cm^3/g$ à 0,8 $cm^3/g$ et une surface spécifique de cuivre allant de 0,5 $m^2/g$ à 10,0 $m^2/g$.

**3.** Procédé selon la revendication 1, **caractérisé en ce que** l'on met en oeuvre des catalyseurs complets de cuivre contenant du $TiO_2$ et présentant une teneur en cuivre, exprimée en CuO, allant de 20% à 60% en poids, une surface spécifique allant de 10 $m^2/g$ à 150 $m^2/g$, une porosité allant de 0,1 $cm^3/g$ à 0,5 $cm^3/g$ et une surface spécifique de cuivre allant de 0,5 $m^2/g$ à 3,0 $m^2/g$.

**4.** Procédé selon la revendication 1, **caractérisé en ce que** l'on met en oeuvre des catalyseurs de cuivre supporté sur $ZrO_2$ qui présentent une teneur en cuivre, exprimée en CuO, allant de 2% à 8% en poids, une surface spécifique allant de 50 $m^2/g$ à 150 $m^2/g$, une porosité allant de 0,2 $cm^3/g$ à 0,4 $cm^3/g$ à une surface spécifique de cuivre allant de 1,0 $m^2/g$ à 3,0 $m^2/g$.

**5.** Procédé selon la revendication 1, **caractérisé en ce que** l'on met en oeuvre des catalyseurs de cuivre supporté sur du charbon actif qui présentent une teneur en cuivre, exprimée en CuO, allant de 2% à 8% en poids, une surface spécifique allant de 500 $m^2/g$ à 1 000 $m^2/g$, une porosité allant de 0,5 $cm^3/g$ à 0,8 $cm^3/g$ et une surface spécifique de cuivre allant de 1,0 $m^2/g$ à 5,0 $m^2/g$.

**6.** Procédé selon la revendication 1, **caractérisé en ce que** l'on élimine les impuretés halogénées dans la phase liquide, en fonctionnement en continu, avec une charge de liquide allant de 0,05 kg à 50 kg de mélange d'ester/L de catalyseur * h, à une température allant de 50°C à 250°C et sous une pression allant de 1 bar à 100 bars.

**7.** Procédé selon la revendication 1, **caractérisé en ce que** l'on élimine les impuretés halogénées dans la phase liquide, en fonctionnement en continu, avec une charge de liquide allant de 0,1 kg à 10 kg de mélange d'ester/L de catalyseur * h, à une température allant de 100°C à 200°C et sous une pression allant de 1 bar à 10 bars.

**8.** Procédé selon la revendication 1, **caractérisé en ce que** l'on élimine les impuretés halogénées dans la phase liquide, en fonctionnement en continu, avec une charge de liquide allant de 0,2 kg à 1,0 kg de mélange d'ester/L de catalyseur * h, à une température allant de 150°C à 180°C et sous une pression allant de 1 bar à 5 bars.

**9.** Procédé selon la revendication 1, **caractérisé en ce que** l'on met en oeuvre, en tant qu'éduit, une fraction d'ester que l'on obtient au moyen

(a) d'une estérification, avec un alcool à faible masse moléculaire, d'un mélange aqueux d'acide dicarboxylique contenant de l'acide adipique, de l'acide 6-hydroxycaproïque et de faibles quantités de 1,4-cyclohexanediols, mélange que l'on obtient, en tant que produit secondaire, lors l'oxydation de cyclohexane en cyclohexanone/cyclohexanol avec de l'oxygène ou des gaz contenant de l'oxygène, et au moyen d'une extraction à l'eau à partir du mélange réactionnel,
(b) de l'élimination, dans une première étape de distillation, de l'alcool en excès et des composés à faible point d'ébullition, et
(c) la séparation du produit de queue, dans une seconde étape de distillation, en une fraction contenant au moins la majeure partie des cyclohexanediols, et en une fraction d'ester destinée à l'hydrogénation et qui est essentiellement exempte de 1,4-cyclohexanediols.

**10.** Procédé selon la revendication 1, **caractérisé en ce que** l'on réduit à moins de 0,1 ppm la teneur en composés halogénés organiques.